# EUROPEAN PATENT APPLICATION

(11) **EP 3 524 320 A1**
(43) Date of publication of application: **14.08.2019**
(21) Application number: 19154594.6
(22) Date of filing: 30.01.2019
(51) Int. Cl.: A61N 5/10

(54) **ION SOURCE DEVICE, PARTICLE BEAM GENERATION, AND ION BEAM GENERATION METHOD**

(30) Priority: 09.02.2018 JP 2018021802
(71) Applicant: National Institutes for Quantum and Radiological Science and Technology, Inage-ku Chiba-shi Chiba 263-8555 (JP)
(72) Inventor: MIZUSHIMA, Kota, Chiba-shi, CHIBA 2638555 (JP); IWATA, Yoshiyuki, Chiba-shi, CHIBA 2638555 (JP); MURAMATSU, Masayuki, Chiba-shi, CHIBA 2638555 (JP); SHIRAI, Toshiyuki, Chiba-shi, CHIBA 2638555 (JP)
(74) Representative: Cabinet Plasseraud

(57) **Abstract**

There are provided a small-sized ion source device, a particle beam generation device, and an ion beam generation method which generate a plurality of ion species while switching the ions species in a short time. An ion source device 1 which generates an ion beam from gas introduced into a vacuum, the ion source device including: a chamber 12 whose inside is a vacuum; a gas supply unit 26 which supplies gas to the inside of the chamber; and an ionization energy supply unit which supplies ionization energy for ionizing atoms of the gas to the inside of the chamber, wherein the gas supply unit 26 includes: a plurality of gas cylinders (17A, 17B, and 17C) each of which is filled with each of a plurality types of gases; pipes (24A, 24B, 24C, and 25) connected to the gas cylinders to feed the gases to the chamber; and pulse-control gas valves (21A, 21B, and 21C) which are attached to the pipes and are settable to an open state in a pulsed manner for a short period of time.

## Description

### TECHNICAL FIELD

The present invention relates to a particle beam generation device for generating a high-energy ion beam (particle beam), an ion source device for generating an ion beam, and an ion beam generation method.

### BACKGROUND ART

Conventionally, there is provided a particle beam therapy device which performs treatment by irradiating an affected area such as cancer cells with charged particles. Examples of charged particles (ion species) used in the particle beam therapy device include hydrogen ions and carbon ions.

Many particle beam therapy devices use only one ion species, but there is disclosed a method in which a combination of a plurality of ion species are irradiated so that therapeutic effect can be increased (see Non Patent Literature 1). Thus, there is a demand for development of a compact particle beam therapy device which can use a plurality of ion species while switching the ion species in a short time.

In this case, there is proposed another type of charged particle beam system, in which two or more of ion species can be used while switching the ion species (see Patent Literature 1). In such a charged particle beam system, a plurality of ion source devices are installed, and the ion source devices are switched depending on the necessary ion species.

However, a particle beam therapy device in which two or more of ion species can be used by being switched has a plurality of ion source devices installed; therefore, the device has a disadvantage that the device is large.

To the contrary, in order to make the particle beam therapy device smaller, a method is proposed. In the method, the depth of an irradiation target and the maximum range in water of each ion species are compared at the time of irradiation, and an ion whose maximum range in water is greater than the depth of the irradiation target is selected and applied to the irradiation target (see Patent Literature 1).

However, the device of Patent Literature 1 also needs a plurality of ion source devices such as an ion source of hydrogen molecules and an ion source of helium, and Patent Literature 1 cannot solve the problem that a plurality of ion source devices make the device larger.

### CITATION LIST

### PATENT LITERATURE

Patent Literature 1: Japanese Patent Application Laid-Open No. 2015-84886

### NON PATENT LITERATURE

Non Patent Literature 1: Taku Inaniwa et al., "Treatment planning of intensity modulated composite particle therapy with dose and linear energy transfer optimization", Phys. Med. Biol. 62 (2017) 5180-5197.

### SUMMARY OF THE INVENTION

### TECHNICAL PROBLEMS

In view of the above problems, an object of the present invention is to provide an ion source device, a particle beam generation device, and an ion beam generation method which can of generate a plurality of ion species while switching the ion species in a short time.

### SOLUTION TO PROBLEMS

The present invention is an ion source device, a particle beam generation device, and an ion beam generation method. The ion source device generates an ion beam from gas introduced into a vacuum and includes: a chamber whose inside is a vacuum; a gas supply unit which supplies gas to the inside of the chamber; and an ionization energy supply unit which supplies ionization energy for ionizing atoms of the gas to the inside of the chamber. The gas supply unit includes: a plurality of gas cylinders each of which is filled with each of a plurality types of gases; a gas passage connected to the gas cylinders to feed the gases to the chamber; and a pulse-control gas valve which is attached to the gas passage and is settable to an open state in a pulsed manner for a short period of time.

### ADVANTAGEOUS EFFECTS OF INVENTION

According to the present invention, it is possible to provide an ion source device, a particle beam generation device, and an ion beam generation method capable of generating a plurality of ion species while switching the ions species in a short time.

### BRIEF DESCRIPTION OF THE DRAWINGS

Fig. 1 is a configuration diagram showing a whole configuration of a particle beam generation device.
Fig. 2 is a configuration diagram showing a configuration of an ion source device.
Fig. 3 is a functional block diagram of the particle beam generation device.
Fig. 4 is a time chart for describing an ion source control method.
Fig. 5 is a partially enlarged view of the time chart for describing the ion source control method.
Fig. 6 is a configuration diagram showing a configuration of an ion source device according to Embodiment 2.

### DESCRIPTION OF EMBODIMENTS

Hereinafter, an embodiment of the present invention will be described with reference to the drawings.

### EMBODIMENT 1

### <Whole configuration of particle beam generation device>

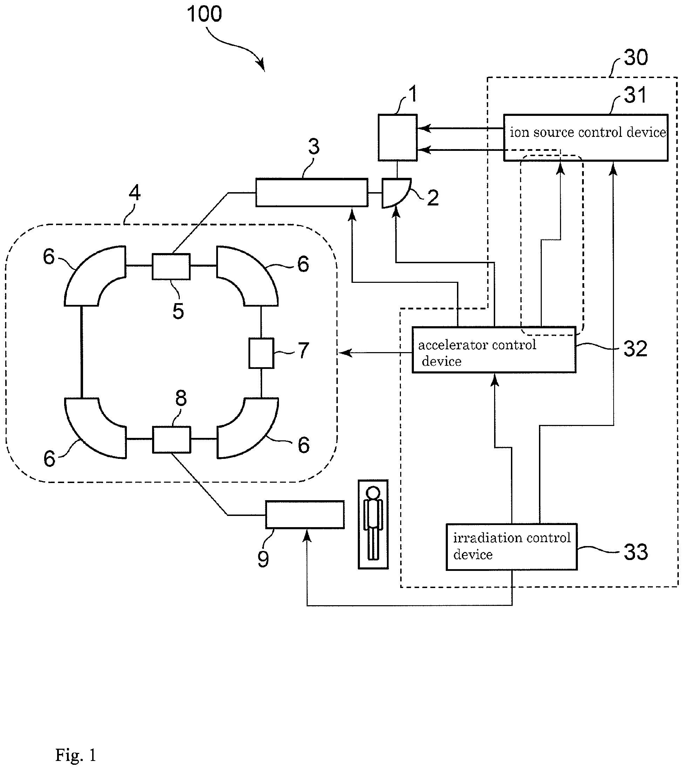

Fig. 1 is a configuration diagram showing a whole configuration of a particle beam generation device 100. The particle beam generation device 100 includes an ion source device 1, an ion separation device 2, a pre-stage accelerator 3, a main accelerator 4, an irradiation device 9, and a control device 30 which controls these devices and accelerators. Note that, although in the illustrated example, an ion source control device 31, an accelerator control device 32, an irradiation control device 33, and an ECR (Electron Cyclotron Resonance) control unit 41 are provided in the control device 30, but the present invention is not limited thereto, and the ion source control device 31, the accelerator control device 32, the irradiation control device 33, and the ECR control unit 41 may be provided separately and may communicate with one another to transmit and receive necessary data.

The ion source device 1 is a device which removes electrons from the atoms of the introduced gas by ionizing the gas introduced into the vacuum by plasma discharge or by irradiating an electron beam and which thus ionizes the gas to generate an ion beam to be ejected in a predetermined direction.

The ion separation device 2 is a device which separates and extracts only target ions from the ion beam generated by the ion source device 1, and is provided with a mass spectrometer (not shown) using a magnetic field. The mass spectrometer has an ion-separation electromagnet (not shown) and a slit (not shown) to separate the ion beam. The slit is provided at an exit of a region to which the magnetic field generated by the ion-separation electromagnet is applied. The value of the current supplied to the ion separation electromagnet is adjusted to adjust the magnetic field so that the ion species (which are target ions) having the target mass and the target ion valence are separated and extracted.

The pre-stage accelerator 3 is a linear accelerator, which is one type of accelerator. The pre-stage accelerator 3 accelerates the ion beam of the target ions separated and extracted by the ion separation device 2 to a predetermined energy by a high-frequency electric field and supplies the ion beam to the main accelerator 4.

The main accelerator 4 is a synchrotron, which is a type of accelerator. The main accelerator 4 receives the ion beam accelerated by the pre-stage accelerator 3 and further accelerates the received ion beam to high energy necessary for treatment and the like. The main accelerator 4 includes: an injection device 5 which injects an ion beam into the main accelerator 4; deflection electromagnets 6 arranged in a circle to deflect the injected ion beam so that the ion beam goes around on the same circular orbit; an acceleration cavity 7 which applies a high-frequency electric field to accelerate the ion beam, and an emission device 8 which extracts the ion beam accelerated to a predetermined high energy from the main accelerator 4. The main accelerator 4 has four operation modes: an injection mode for receiving injection of an ion beam in a standby state; an acceleration mode for accelerating the injected ion beam; an emission mode for emitting the accelerated ion beam; and a deceleration mode for returning the state to a standby state, and an operation is performed by repeating these four operation modes in this order.

The irradiation device 9 is a device which irradiates a target tumor or the like of a patient with the high-energy ion beam taken out from the main accelerator 4. The irradiation device 9 includes an X direction scanning magnet and a Y direction scanning magnet which adjust a position of the ion beam in the XY direction (directions perpendicular to an irradiation direction of the ion beam), a range shifter which finely adjusts a stop position of the irradiation spot (irradiation depth) in the Z direction (the traveling direction of the ion beam) of the ions in the ion beam, and a scanning monitor which measures an irradiation dose of the ion beam for each irradiation spot.

The control device 30 includes the ion source control device 31, the accelerator control device 32, and the irradiation control device 33. The ion source control device 31 controls an operation of the ion source device 1. The accelerator control device 32 controls operations of the ion separation device 2, the pre-stage accelerator 3, and the main accelerator 4. The irradiation control device 33 controls the three-dimensional position of the irradiation spot of the ion beam irradiated by the irradiation device 9 while monitoring the irradiation dose of the applied ion beam and the like. In addition, the irradiation control device 33 transmits requests such as the energy (acceleration voltage) and intensity of the ion beam and the change of ion species to be irradiated to the ion source control device 31 and the accelerator control device 32 according to the irradiation depth. Further, the accelerator control device 32 transmits a gas introduction instruction to the ion source control device 31 so that the ion beam is injected in synchronism with the timing of the injection mode of the main accelerator 4.

### <Details of ion source device>

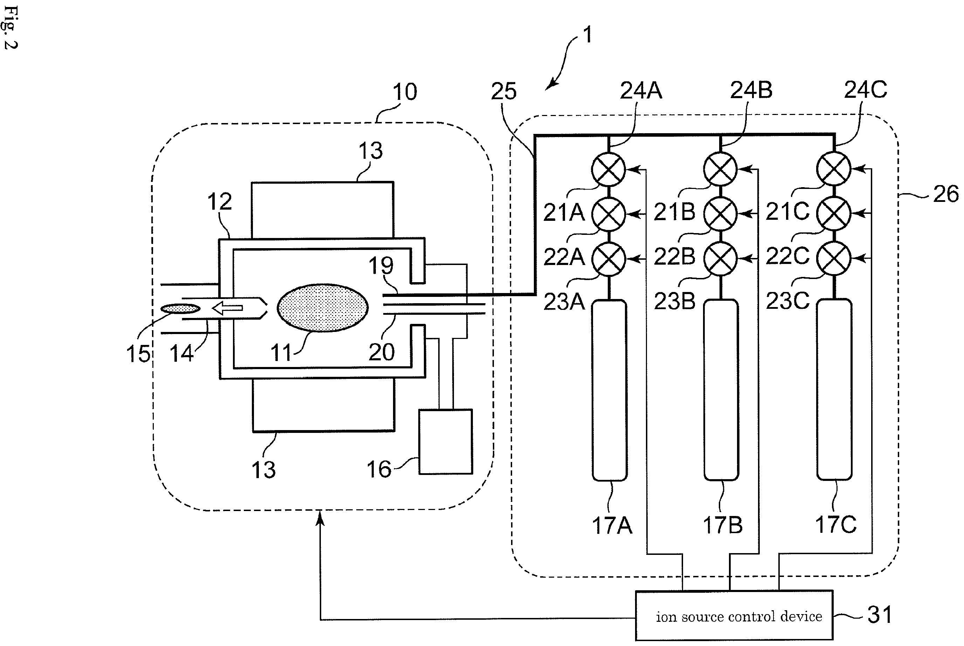

Fig. 2 is a configuration diagram showing the configuration of the ion source device 1. For the ion source device 1, some methods can be used to generate an ion beam, and the methods are different in how to supply ionization energy for ionizing gas atoms.

In this example, an ECR ion source 10 is used which ionizes gas atoms by an ECR discharge method. The ECR ion source 10 employs a principle in which microwave power is externally applied to the gas introduced into a vacuum to ionize electrons which trigger plasma discharge, the ionized electrons are then confined by a magnetic field so that the ionized electrons repeatedly resonate with a microwave and thus ionize the gas atoms.

The ECR ion source 10 is a substantially cylindrical body whose inside is hollow and is laterally laid, and has a plasma chamber 12 made of a vacuum material.

The plasma chamber 12 is provided with: a gas nozzle 19, on one side (on the right side in the drawing), for introducing gas; an electrode 14 for ejecting an ion beam 15 on the other side (the left side in the drawing); and an electromagnet 13 to surround the outer periphery in a supply and ejection direction that is the direction from the one side to the other side.

A vacuum port 18a is provided on the one side (the right end in the drawing) of the plasma chamber 12. In the vacuum port 18a are inserted the gas nozzle 19 for introducing gas into a central part of the inside of the plasma chamber 12 and a microwave waveguide 20 (ionization energy supply unit) which is connected to a microwave power source and transmits a microwave into the plasma chamber 12. The vacuum port 18a is covered with a vacuum flange which covers the vacuum port 18a from outside, and a vacuum pump 16 is connected to the vacuum flange through a vacuum pipe. By this arrangement, the inside of the plasma chamber 12 is kept a vacuum.

On the other side (the left end in the drawing) of the plasma chamber 12, there is provided an ejection port 18b which ejects the ions generated in the plasma chamber 12 to outside of the plasma chamber 12. In addition, the ejection port 18b is provided with an electrode 14 for pulling the ions in the plasma chamber 12 and for extracting the ions from the ejection port 18b.

In the ECR ion source 10, gas is introduced from the gas nozzle 19 into the plasma chamber 12 which is kept a vacuum by the vacuum pump 16, microwave power is applied from the microwave waveguide 20 to the gas introduced into the plasma chamber 12, electrons serving as a trigger of plasma discharge are ionized, the ionized electrons are confined by the magnetic field of the electromagnet 13 surrounding the side surface of the plasma chamber 12, and the ions repeatedly resonate with the microwave so that plasma 11 is generated, thereby ionizing the atoms of the gas. Then, the ions generated in the plasma chamber 12 are extracted from the plasma chamber 12 by the electrode 14, and the ions are given a predetermined energy and are ejected in a predetermined direction as the ion beam 15.

The above-described series of operations on the ECR ion source 10 is controlled by the ion source control device 31.

It should be noted that the ion source device 1 is not limited to the above-described system of the ECR ion source 10, and a system such as a PIG (Penning Ionization Gauge) ion source or an EBIS (Electron Beam Ion Source) may also be used. For example, the PIG type ion source has a structure in which cathodes are arranged at the both ends of a cylindrical anode, and a strong magnetic field is applied in the axial direction, and the PIG type ion source uses a principle that electrons are emitted from the cathodes and are confined in the radial direction by the magnetic field and in the axial direction by the cathodes at the both ends to generate high density plasma. In addition, the EBIS (Electron Beam Ion Source) uses a principle that a high energy electron beam is made to repeatedly collide with the same atoms (ions) so that the atoms are ionized.

The ion source device 1 includes a gas supply unit 26 which supplies gas to the ECR ion source 10.

The gas supply unit 26 includes a plurality of gas supply systems each for supplying each of a plurality of types of gases to the ECR ion source 10, and in this example, the gas supply unit 26 includes three types of gas supply systems of an oxygen (O₂) gas supply system, a methane (CH₄) gas supply system, and a helium (He) gas supply system.

For example, the oxygen gas supply system includes an oxygen gas cylinder 17A filled with oxygen gas and an oxygen gas supply pipe 24A (gas passage) one end of which is connected to the oxygen gas cylinder 17A and which supplies the oxygen gas to the ECR ion source 10. The oxygen gas supply pipe 24A is provided with an oxygen gas pulse-control gas valve 21A, an oxygen gas shut-off gas valve 22A, and an oxygen gas flow-rate-adjustment gas valve 23A.

The oxygen gas shut-off gas valve 22A can shut off the supply of the oxygen gas passing through the oxygen gas supply pipe 24A, and the oxygen gas flow-rate-adjustment gas valve 23A adjusts a flow rate of oxygen gas passing through the oxygen gas supply pipe 24A.

The oxygen gas pulse-control gas valve 21A is a gas valve of high speed response, and a valve using a piezoelectric element or a solenoid valve is used, for example. The oxygen gas pulse-control gas valve 21A can be switched between a closed state and an open state quickly, and can be opened for a short time in a pulsed manner. The oxygen gas pulse-control gas valve 21A preferably has an open pulse width of 1 ms or less, more preferably 100 µs or less, and further preferably 10 µs or less. It should be noted that the expression "in a pulsed manner" means that the valve is in an ON state only for a minute time such as 1 ms or less, 100 µs or less, or 10 µs or less and is in an OFF state before and after the ON state.

Similarly, the methane gas supply system has a methane gas cylinder 17B, a methane gas supply pipe 24B (gas passage), a methane gas pulse-control gas valve 21B, a methane gas shut-off gas valve 22B, and a methane gas flow-rate-adjustment gas valve 23B. The helium gas supply system has a helium gas cylinder 17C, a helium gas supply pipe 24C (gas passage), a helium gas pulse-control gas valve 21C, a helium gas shut-off gas valve 22C, and a helium gas flow-rate-adjustment gas valve 23C.

Note that the gas cylinders (17B and 17C), the gas supply pipes (24B and 24C), and the gas valves (21B, 22B, 23B, 21C, 22C, and 23C) constituting the methane gas supply system and the helium gas supply system have the same configurations and functions as the corresponding gas cylinder (17A), gas supply pipe (24A), and various gas valves (21A, 22A, and 23A) of the oxygen gas supply system, and the description thereof is omitted.

The other end of each of the oxygen gas supply pipe 24A, the methane gas supply pipe 24B, and the helium gas supply pipe 24C is connected to a gas introduction pipe 25 (gas passage) to one end of which a gas nozzle 19 is connected.

The ion source control device 31 controls opening/closing switching and adjustment of flow rate by various gas valves (21A, 22A, 23A, 21B, 22B, 23B, 21C, 22C, and 23C) of the oxygen gas supply system, the methane gas supply system and the helium gas supply system.

### <Functional blocks of particle beam generation device>

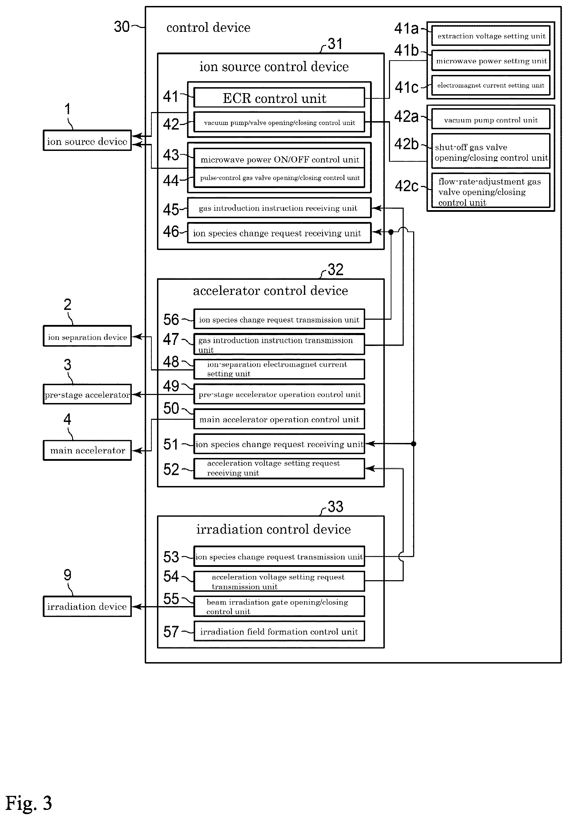

Fig. 3 is a functional block diagram of the particle beam generation device 100. As described above, the particle beam generation device 100 includes, as the control device 30, the ion source control device 31, the accelerator control device 32, and the irradiation control device 33.

### <Function blocks of ion source control device>

The ion source control device 31 functions as an ECR control unit 41, a vacuum pump/valve opening/closing control unit 42, a microwave power ON/OFF control unit 43, a pulse-control gas valve opening/closing control unit 44, a gas introduction instruction receiving unit 45, and an ion species change request receiving unit 46.

The function of the ECR control unit 41 is further subdivided to function as an extraction voltage setting unit 41a, a microwave power setting unit 41b, and an electromagnet current setting unit 41c.

In the ECR ion source 10, the electromagnet current setting unit 41c selectively sets the frequency of the electrons circularly moving around the magnetic field by the electromagnet 13 to a value close to the frequency of the microwave applied through the microwave waveguide 20, and sets the current value supplied to the electromagnet 13 (a value of electromagnet current for confining magnetic field) to such a current that the plasma 11 is easily generated in the plasma chamber 12 by the electron cyclotron resonance (ECR) phenomenon.

With respect to the microwave power source of the ECR ion source 10, the microwave power setting unit 41b performs setting of the microwave power (input power for acceleration electric field) to be input into the plasma chamber 12 through the microwave waveguide 20. This setting can change the density of the plasma 11 generated in the plasma chamber 12.

In the ECR ion source 10, the extraction voltage setting unit 41a performs setting of a predetermined extraction voltage to be applied to the electrode 14 in order to extract the target ions, with a predetermined energy, from the plasma 11 generated in the plasma chamber 12. In this case, Table 1 shows examples of the settings of the extraction voltages and other conditions depending on ion species.

**[Table 1]**

| | Trivalent oxygen | Divalent carbon | Divalent helium |
|---|---|---|---|
| Extraction voltage [kV] | 42.67 | 48.00 | 16.00 |
| Input power for acceleration electric field [W] | 400 | 330 | 250 |
| Value of electromagnet current for confining magnetic field [A] (magnetic field [T]) | 865A (1.20T) | 810A (1.10T) | 840A (1.16T) |
| Value of ion-separation electromagnet current [A] (magnetic field [T]) | 267A (0.80T) | 300A (0.90T) | 100A (0.30T) |

Table 1 shows an example of the extraction voltage, the input power for acceleration electric field, the value of electromagnet current for confining magnetic field (magnetic field) which are respectively set by the extraction voltage setting unit 41a, the microwave power setting unit 41b, and the electromagnet current setting unit 41c when trivalent oxygen ions, divalent carbon ions, and divalent helium ions are extracted at a per-nucleon power of 8 keV/n in the ECR ion source 10.

The function of the vacuum pump/valve opening/closing control unit 42 is further subdivided to function as a vacuum pump control unit 42a, a shut-off gas valve opening/closing control unit 42b, and a flow-rate-adjustment gas valve opening/closing control unit 42c.

The vacuum pump control unit 42a controls an operation of the vacuum pump 16 so that the inside of the plasma chamber 12 is kept at high vacuum.

For example, when the target ions are the trivalent oxygen ions, the shut-off gas valve opening/closing control unit 42b performs control such that only the oxygen gas shut-off gas valve 22A of the oxygen gas supply system is opened and that the other methane gas shut-off gas valve 22B of the other methane gas supply system and helium gas shut-off gas valve 22C of the helium gas supply system are closed (see Fig. 2). By this control, only the source gas for generating the target ions can be supplied to the ECR ion source 10.

Similarly, for example, when the target ions are the trivalent oxygen ions, the flow-rate-adjustment gas valve opening/closing control unit 42c controls a degree of opening of the oxygen gas flow-rate-adjustment gas valve 23A of the oxygen gas supply system, so that it is possible to adjust a flow rate of the oxygen gas flowing through the oxygen gas supply pipe 24A.

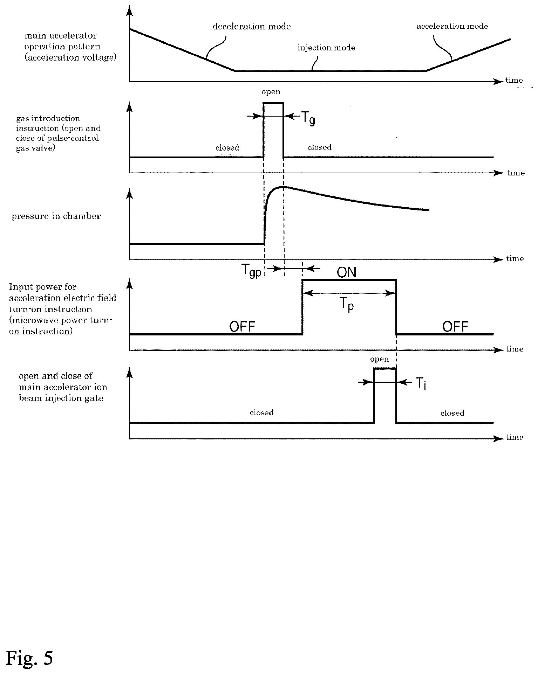

The microwave power ON/OFF control unit 43 (ionization energy supply-period control unit) controls (ON/OFF control) the output of the microwave power (input power for acceleration electric field) having been set, with respect to the microwave power source of the ECR ion source 10. The microwave power ON/OFF control unit 43 turns on the microwave power at a predetermined timing which is after the time of opening or closing, of the gas valve, by the pulse-control gas valve opening/closing control unit 44 and which is an appropriate timing for generating the ions of the ion species received from an ion species change request transmission unit 56 of the accelerator control device 32, and the microwave power ON/OFF control unit 43 turns off the microwave power after a predetermined time has elapsed. Note that, in the example of Fig. 5, the predetermined timing for turning on the microwave power is determined with respect to the time when the gas valve is closed, but the predetermined timing may be determined with respect to the time when the gas valve is opened.

For example, when the target ions are trivalent oxygen ions, the pulse-control gas valve opening/closing control unit 44 performs control such that the oxygen gas pulse-control gas valve 21A opens and closes in a pulsed manner. By this control, the oxygen gas pulse-control gas valve 21A can be switched quickly from the closed state to the open state and return to the original closed state, so that the oxygen gas pulse-control gas valve 21A can be opened for a short period of time in a pulsed manner. This makes it possible to adjust, to a necessary minimum, the supply amount of the source gas to be supplied to the ECR ion source 10 to generate the target ions, and it is also possible to supply the source gas in a short time.

The gas introduction instruction receiving unit 45 receives a gas introduction instruction transmitted by the accelerator control device 32 to inject the ion beam in synchronism with the timing of the injection mode of the main accelerator 4.

The ion species change request receiving unit 46 receives an ion species change request transmitted from the irradiation control device 33 when the ion species (target ions) of the ion beam emitted by the irradiation device 9 is changed, and receives an ion species change request which is a trigger transmitted by the accelerator control device 32 in synchronism with the timing of switching from the emission mode to the deceleration mode of the main accelerator 4.

### <Function blocks of accelerator control device>

The accelerator control device 32 functions as the ion species change request transmission unit 56, a gas introduction instruction transmission unit 47, an ion-separation electromagnet current setting unit 48, a pre-stage accelerator operation control unit 49, a main accelerator operation control unit 50, an ion species change request receiving unit 51, and an acceleration voltage setting request receiving unit 52.

The ion species change request transmission unit 56 transmits an ion species change request serving as a trigger to the ion source control device 31 in synchronism with the timing of switching from the emission mode of the main accelerator 4 to the deceleration mode.

The gas introduction instruction transmission unit 47 transmits a gas introduction instruction to the ion source control device 31 so that the ion beam is injected in synchronism with the timing of the injection mode of the main accelerator 4.

In the ion separation device 2, in order to separate and extract the ion species (target ions) having a target mass and ion valence from the ion beam, the ion-separation electromagnet current setting unit 48 controls the magnetic field applied to the ion beam by setting the value of the current supplied to the ion separation electromagnet in accordance with the target ions.

The pre-stage accelerator operation control unit 49 controls the high-frequency electric field of the pre-stage accelerator 3 such that the ion beam of the target ions separated and extracted by the ion separation device 2 are accelerated to a predetermined energy.

The main accelerator operation control unit 50 controls the individual operation in each of the four operation modes (the injection mode, the acceleration mode, the emission mode, and the deceleration mode) of the main accelerator 4, and performs control such that the four operation modes are sequentially operated in this order. Note that the period of one cycle from the injection mode to the deceleration mode is several seconds to several tens of seconds. As a result, the ion beam of the target ions repeatedly entering from the pre-stage accelerator 3 are accelerated to high energy necessary for treatment or the like each time.

The ion species change request receiving unit 51 receives an ion species change request transmitted by the irradiation control device 33 when the ion species (target ions) of the ion beam irradiated by the irradiation device 9 is changed.

The acceleration voltage setting request receiving unit 52 receives an acceleration voltage setting request transmitted by the irradiation control device 33 when the irradiation device 9 changes the energy (acceleration voltage) or the intensity of the ion beam according to the irradiation depth.

### <Function blocks of irradiation control device>

The irradiation control device 33 functions as an ion species change request transmission unit 53, an acceleration voltage setting request transmission unit 54, a beam irradiation gate opening/closing control unit 55, and an irradiation field formation control unit 57.

When changing the ion species (target ions) of the ion beam irradiated by the irradiation device 9, the ion species change request transmission unit 53 transmits an ion species change request to the ion source control device 31 and the accelerator control device 32.

When the irradiation device 9 changes the energy (acceleration voltage) or intensity of the ion beam according to the irradiation depth, the acceleration voltage setting request transmission unit 54 transmits an acceleration voltage setting request to the accelerator control device 32.

The beam irradiation gate opening/closing control unit 55 controls the opening and closing of a gate, which is the ejection port of the ion beam irradiated by the irradiation device 9. By this control, it is possible to control the irradiation dose at the three-dimensional position of the irradiation spot of each of the ion beams irradiated by the irradiation device 9.

In addition, the irradiation field formation control unit 57 controls a deflection angle of the ion beam irradiated by the irradiation device 9. By this control, the irradiation device 9 can control the irradiation position of the ion beam in accordance with each irradiation spot.

### <Operation of particle beam generation device>

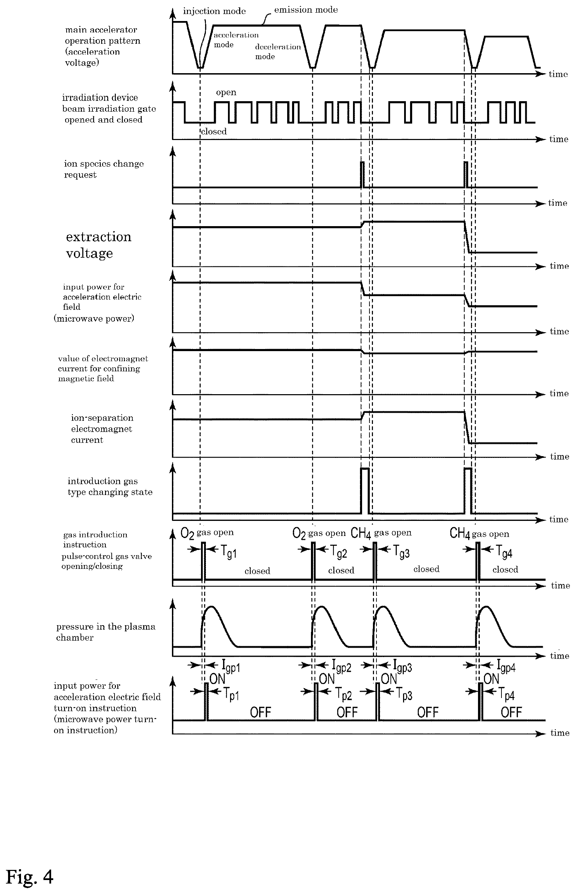

Fig. 4 is a time chart for describing the operation of the particle beam generation device 100.

In this case, an example is described in which the irradiation device 9 provides, as ion beams, irradiation of trivalent oxygen ions (by oxygen gas) twice, then divalent carbon ions (by methane gas) once, lastly, divalent helium ions (by helium gas) once.

The irradiation device 9 provides irradiation of an ion beam in synchronism with a series of operation modes from the injection mode to the deceleration mode, which are repeatedly operated by the main accelerator 4. Each irradiation of the ion beam by the irradiation device 9 is performed in the emission mode of the main accelerator 4 and is performed by opening a beam irradiation gate of the irradiation device 9 for a predetermined period of time (opening period) intermittently for a few times with a predetermined period between emissions. The opening time in this operation is set to a range from several milliseconds to several seconds. By this operation, the intensity of the ion beam to be irradiated is adjusted.

As shown in the drawing, while a main accelerator operation pattern is in the emission mode, the irradiation device beam irradiation gate is opened and closed, and change of ion species is required after the irradiation device beam irradiation gate is closed and before the next injection mode is started.

In addition, after the change of ion species is required and before the injection mode is started, the extraction voltage, the input power for acceleration electric field, the electromagnetic current for the confining magnetic field, and the ion separation electromagnet current are set to the values corresponding to the next ion species.

In addition, after the change of the ion species is required and until a time period necessary for changing introduced gases has elapsed, a status is set to an introduction gas type changing state so that a gas introduction instruction is not issued until the status becomes not the introduction gas type changing state.

The "gas introduction instruction", "chamber vacuum degree", "input power for acceleration electric field turn-on instruction" shown in the lower part of Fig. 4 are on (open) for an extremely short time period (at a magnitude of, for example, less than or equal to 1/10 or 1/100) compared to the other items. For this reason, in the drawing, the time axis of the part in the on (open) state shown in an enlarged manner, and the part in the off (closed) state is shown being partially cut. Actually, this part is shown with the time axis shown in Fig. 5.

### <<Ion beam generation operation>>

Fig. 5 is a partially enlarged view of a time chart for describing in detail the operation shown in Fig. 4.

The ion source device 1 generates an ion beam such that the ion beam can enter the main accelerator 4 when the main accelerator 4 is in the injection mode.

The operation modes of the ion source device 1 include a standby mode in an idling state, a condition setting mode for setting a condition for generating an ion beam of target ions, and a generation mode for actually generating an ion beam.

### [Standby mode]

In the standby mode, on the ion source device 1, under the control of the ion source control device 31 (the vacuum pump/valve opening/closing control unit 42), the vacuum pump 16 is activated, and the plasma chamber 12 is being vacuumed.

### [Condition setting mode]

The transition from the standby mode to the condition setting mode is started when the ion source control device 31 receives an ion species change request (in this case, ion species setting request) transmitted from the irradiation control device 33. The ion species setting request in this case, for example, requires that trivalent oxygen ions are set as target ions (ion species).

Note that the ion species setting request transmitted by the irradiation control device 33 is also transmitted to the accelerator control device 32.

In the ion source device 1, under the control of the ion source control device 31 (the vacuum pump/valve opening/closing control unit 42) having received the ion species setting request for trivalent oxygen ions, the oxygen gas shut-off gas valve 22A is switched from the closed state to the open state, and the degree of opening of the oxygen gas flow-rate-adjustment gas valve 23A is adjusted to a predetermined value. By this operation, if the remaining oxygen gas pulse-control gas valve 21A is opened, the gas supply unit 26 gets in a state for supplying an oxygen gas, in which state, an oxygen gas can be supplied at a predetermined flow rate from the oxygen gas cylinder 17A into the plasma chamber 12 via the oxygen gas supply pipe 24A, the gas introduction pipe 25, and the gas nozzle 19.

In addition, in the ion source device 1, under the control of the ion source control device 31 (the ECR control unit 41) having received the ion species setting request for trivalent oxygen ions, the predetermined value of the current to be supplied to the electromagnet 13 (the value of electromagnet current for confining magnetic field) for confining the trivalent oxygen ions in the plasma chamber 12, the predetermined microwave power to be input from the microwave power source to the plasma chamber 12, and the predetermined extraction voltage to be applied to the electrode 14 are set (see Table 1). Then, the current of the current value having been set is supplied to the electromagnet 13, and the set extraction voltage is applied to the electrode 14. However, at this point in time, the microwave power source merely sets the predetermined microwave power just to be ready, and the microwave power is not output into the plasma chamber 12.

### [Generation mode]

Next, the transition from the condition setting mode to the generation mode is started by the ion source control device 31 receiving a gas introduction instruction transmitted from the accelerator control device 32. The accelerator control device 32 transmits the gas introduction instruction to the ion source control device 31 (see Fig. 5) in order to inject the ion beam in synchronism with the timing of the injection mode of the main accelerator 4.

Since the accelerator control device 32 has previously received the ion species setting request (in this case, it is requested to set trivalent oxygen ions as the target ions (ion species)) transmitted from the irradiation control device 33, the accelerator control device 32 transmits to the ion source control device 31 a gas introduction instruction to introduce oxygen gas.

In the ion source device 1, under the control of the ion source control device 31 (the pulse-control gas valve opening/closing control unit 44) having received the gas introduction instruction, the oxygen gas pulse-control gas valve 21A opens and closes in a pulsed manner. Note that, in this case, a pulse width Tg when the open state is maintained is preferably 1 ms or less, more preferably 100 µs or less, and still more preferably 10 µs or less. With this arrangement, a small amount of oxygen gas can be supplied into the plasma chamber 12. At this time, the pressure inside the plasma chamber 12 rapidly rises simultaneously with the start of the supply of the oxygen gas, then turns to falling and gradually decreases. The supply amount of the oxygen gas into the plasma chamber 12 is adjusted to be small, and the oxygen gas is supplied in a short time so that the degree of vacuum in the plasma chamber 12 returns to the degree of vacuum before the oxygen gas has been supplied, within the time period of one cycle from the injection mode to the deceleration mode which are operated by the main accelerator 4, or within 2 seconds preferably within 1 second.

In the ion source device 1, after the opening and closing operations, in a pulsed manner, of the oxygen gas pulse-control gas valve 21A are completed under the control of the ion source control device 31 (the microwave power ON/OFF control unit 43) and then after a short time period of interval Igp elapses, the microwave power source outputs the previously set predetermined microwave power (the input power for acceleration electric field) in the plasma chamber 12. It is preferable that the interval Igp be set to the period from the time when the pressure caused by the oxygen gas in the plasma chamber 12 turns from rising to falling and to the time when the pressure starts to decrease steadily. By this, since the microwave power is output when the oxygen gas amount in the plasma chamber 12 is high and stable, it is possible to stably generate a high density plasma 11. In addition, the output time Tp of the microwave power (the input power for acceleration electric field) is set to the period whose end limit is a time when the amount of oxygen gas in the plasma chamber 12 decreases too low for the plasma 11 to be stably generated. That is, the output time Tp is set to a period Tc from the time when the gas amount in the chamber starts to increase and to the time when the chamber becomes a vacuum, in other words, to a period in which a gas amount changing curve G1 exists and which is suitable for ion generation. By this, a large amount of plasma 11 can be generated. Concurrently, a large amount of trivalent oxygen ions (target ions) can be generated in the plasma 11.

The trivalent oxygen ions in the plasma 11 generated in the plasma chamber 12 are extracted from the plasma chamber 12 by the electrode 14 and are then converted into the ion beam 15, and the ion beam 15 is ejected from the ion source device 1 and enters the main accelerator 4 through the ion separation device 2 and the pre-stage accelerator 3.

The operation in the generation mode in the ion source device 1 is completed by the above step, and the operation goes back to the condition setting mode for trivalent oxygen ions. At that time, various settings are maintained as they are.

In the ion separation device 2, the magnetic field to be applied to the ion beam is controlled by setting the value of the current to be supplied to the ion separation electromagnet in accordance with the trivalent oxygen ions (target ions) under the control of the accelerator control device 32 (the ion-separation electromagnet current setting unit 48) having received the ion species setting request.

In addition, in the main accelerator 4, an ion beam injection gate is opened for an opening time Ti in synchronism with the timing when the ion beam enters through the pre-stage accelerator 3 in the final stage of the injection mode under the control of the accelerator control device 32 (main accelerator operation control unit 50) so that the entering ion beam of trivalent oxygen ions are received. In this case, the opening time Ti is determined based on the required amount of trivalent oxygen ions required in the ion species setting request transmitted by the irradiation control device 33 to the accelerator control device 32.

Note that in the case where the ion beam of the same trivalent oxygen ions will be generated, it is only necessary to transition again from the maintained condition setting mode for trivalent oxygen ions to the above-mentioned generation mode for trivalent oxygen ions (see Fig. 4).

### <<Ion species changing operation>>

In the section of "ion beam generation operation" above, a description is given on the operation of generating an ion beam of trivalent oxygen ions. Next, a description will be given on the operation in the case where the ion species are changed, for example, from trivalent oxygen ions to divalent carbon ions (see Fig. 4).

The change of ion species is started by the irradiation control device 33 transmitting an ion species change request (in this case, a change request from trivalent oxygen ions to divalent carbon ions) to the ion source control device 31 and the accelerator control device 32.

When the main accelerator 4 transitions from the emission mode to the deceleration mode after the accelerator control device 32 receives the ion species change request transmitted from the irradiation control device 33, the accelerator control device 32 transmits an ion species change request serving as a trigger to the ion source control device 31.

When the ion source control device 31 receives the ion species change request serving as a trigger from the accelerator control device 32 in addition to the ion species change request from the irradiation control device 33, the ion source control device 31 starts to change the ion source device 1 from the condition setting mode for trivalent oxygen ions to the condition setting mode for divalent carbon ions.

It should be noted that the ion source control device 31 performs control such that the ion source device 1 does not transition to the generation mode, by setting to an introduction gas type changing state a certain period after the reception of the ion species change request serving as a trigger transmitted by the accelerator control device 32.

### [Switching of condition setting mode]

When the mode is made to transition from the condition setting mode for trivalent oxygen ions to the condition setting mode of divalent carbon ions, the settings set in the condition setting mode for trivalent oxygen ions are all reset. Then, setting for divalent carbon ions in the condition setting mode is again performed in the same manner as in the above-mentioned case of setting for the trivalent oxygen ions.

That is, in the ion source device 1, under the control of the ion source control device 31 (the vacuum pump/valve opening/closing control unit 42) having received the ion species change request for divalent carbon ions, the methane gas shut-off gas valve 22B of the gas supply unit 26 is switched from the closed state to the open state, and the degree of opening of the methane gas flow-rate-adjustment gas valve 23B is adjusted to a predetermined value.

In addition, in the ion source device 1, under the control of the ion source control device 31 (the ECR control unit 41) having received the ion species change request for divalent carbon ions, the following conditions are set: the predetermined value of the current to be supplied to the electromagnet 13 (the value of electromagnet current for confining magnetic field) for confining the divalent carbon ions in the plasma chamber 12; the predetermined microwave power to be input from the microwave power source to the plasma chamber 12; and the predetermined extraction voltage to be applied to the electrode 14 (see Table 1). Then, the current of the current value having been set is supplied to the electromagnet 13, and the set extraction voltage is applied to the electrode 14.

In the ion separation device 2, the magnetic field to be applied to the ion beam is controlled by setting the value of the current to be supplied to the ion separation electromagnet in accordance with divalent carbon ions under the control of the accelerator control device 32 (the ion-separation electromagnet current setting unit 48) having received the ion species change request.

### [Generation mode after switching of condition setting mode]

Transition to the generation mode is started by the ion source control device 31 receiving, out of the period of the above-mentioned introduction gas type changing state, the gas introduction instruction of a methane gas transmitted by the accelerator control device 32 so as to inject the ion beam into the main accelerator 4 in synchronism with the timing of the injection mode.

In the ion source device 1, under the control of the ion source control device 31 (the pulse-control gas valve opening/closing control unit 44) having received the gas introduction instruction, the methane gas pulse-control gas valve 21B opens and closes in a pulsed manner. By this operation, the pressure in the plasma chamber 12 is sufficiently lowered before a methane gas is supplied, and the pressure rapidly increases simultaneously with the start of the supply, and then turns to falling and gradually decreases. Note that the supply amount of the methane gas into the plasma chamber 12 is adjusted to be small so that the degree of vacuum in the plasma chamber 12 returns to the degree of vacuum before the methane gas has been supplied, within the time period of one cycle from the injection mode to the deceleration mode which are operated by the main accelerator 4, or within 2 seconds preferably within 1 second.

In the ion source device 1, after the opening and closing operations, in a pulsed manner, of the methane gas pulse-control gas valve 21B are completed under the control of the ion source control device 31 (the microwave power ON/OFF control unit 43) and then after a short time period of interval Igp elapses, the microwave power source outputs the previously set predetermined microwave power (the input power for acceleration electric field) in the plasma chamber 12, thereby generating plasma 11.

The divalent carbon ions in the plasma 11 generated in the plasma chamber 12 are extracted from the plasma chamber 12 by the electrode 14 and are then converted into the ion beam 15, and the ion beam 15 is ejected from the ion source device 1 and enters the main accelerator 4 through the ion separation device 2 and the pre-stage accelerator 3.

The operation in the generation mode, in the ion source device 1, of divalent carbon ions is completed by the above step, and the operation goes back to the condition setting mode. At that time, various settings are maintained as they are.

In the above-described section of "ion species changing operation", a description is given on an example of the operation in the case of changing the ion species from trivalent oxygen ions to divalent carbon ions; however, in a similar manner, the ion species can be changed from divalent carbon ions to divalent helium ions.

With the above configuration and operations, it is possible to supply an arbitrary gas out of a plurality of types of gases into the plasma chamber 12, and to generate ions of a type corresponding to the type of the supplied gas. In addition, there is no need for a plurality of ECR ion sources 10 (plasma chambers 12) to generated a plurality of types of ion species; therefore, it is possible to prevent the ion source device 1 and the particle beam generation device 100 from becoming larger.

In addition, the gas supply unit 26 includes the pulse-control gas valves (21A, 21B, and 21C), each of which can make a plurality of types of gases open for a short time (1 ms or less) in a pulsed manner.

This arrangement can supply a small amount of gas into the plasma chamber 12 in a short time. With this arrangement, the pressure in the plasma chamber 12 is reduced back to a low value (initial value) before the gas has been supplied, within a time period (several seconds to several tens seconds) of one cycle from the injection mode to the deceleration mode, which cycle is repeatedly operated by the main accelerator 4 That is, at the timing when gas is supplied into the plasma chamber 12, the pressure in the plasma chamber 12 can always be returned almost to the initial value. Therefore, a plurality of types of gases can be supplied while being switched in a short time. As a result, the ion source device 1 having the gas supply unit 26 can generate a plurality of types of ion species while the ion species being switched in a short time, and it is possible to generate necessary ion species as needed. In addition, since the pulse-control gas valves (21A, 21B, and 21C) are small components, the ion source device 1 is not increased in size because these valves are provided.

The microwave power source (the ionization energy supply unit) is configured to start supplying ionization energy (microwave power) into the plasma chamber 12 after the pulse-control gas valve transitions from the open state to the closed state.

By this operation, the microwave power can be applied in the situation where the pressure in the plasma chamber 12 rapidly is once increased by the gas supplied by the opening and closing operations of the pulse-control gas valve, then slowly turns to falling, and is stable. Therefore, even if the amount of supplied gas is small, stable plasma discharge can be caused. In addition, plasma discharge is started in the state where the amount of gas in the plasma chamber 12 is relatively large, so that high density plasma 11 is generated. As a result, target ions can be generated in the plasma 11 in a high concentration and in a large amount.

The accelerator control device 32 transmits a gas introduction instruction to supply the gas into the plasma chamber 12 of the ion source device 1 in synchronism with a predetermined injection timing so that the ion beam enters the main accelerator 4 at a predetermined timing. The ion source control device 31 receives the gas introduction instruction and performs control on the basis of the gas introduction instruction such that the pulse-control gas valve (21A, 21B, and 21C) of the ion source device 1 is opened for only a short time in a pulsed manner.

Therefore, the main accelerator 4 operates in conjunction with the ion source device 1, and it is possible to certainly inject all or a large part of the target ions generated in the ion source device 1 into the main accelerator 4. As a result, the generated target ions and the gas supplied for the generation are less wasted.

In addition, instead of discharging gas after generating ions by plasma discharge while the plasma chamber 12 is filled with the gas, the gas is supplied in a pulsed manner when the gas in the plasma chamber 12 is suctioned by the vacuum pump 16, the change, in the gas amount in the plasma chamber 12, due to the supply of the gas in a pulsed manner (the gas amount changing curve G 1) is considered, plasma discharge is caused to generate ions at an appropriate timing (output time Tp) during the change in the gas amount, so that it is possible to evacuate the gas in the plasma chamber 12 in a short time while generating necessary ions.

Therefore, it is possible to shorten the time to change the type of gas and to generate different ions. In addition, since the gas used for ion generation is sufficiently evacuated from the inside of the plasma chamber 12 in a short time, it is possible to avoid a state where elements heavier than the ionized element are mixed as gases. As a result, it is possible to avoid the situation where the confinement time of light ions is shortened and where it is difficult to increase the intensity of the ions having a large valence number, and the residual gas can be sufficiently exhausted in a short time so that the intensity of the ions having a large valence number can be increased.

In addition, when ions obtained by a single gas supply are insufficient, it is possible to supply again the same gas into the plasma chamber 12 and to cause plasma discharge to generate the same ions. By this, even when a large amount of ions are required, it is possible to cope with the requirement well.

### EMBODIMENT 2

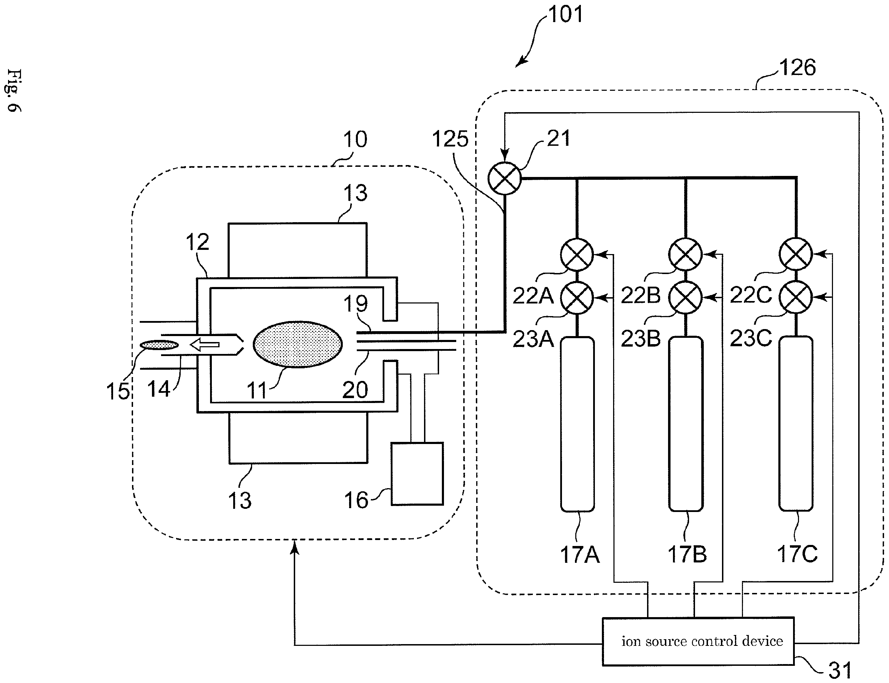

Fig. 6 is a configuration diagram showing a configuration of an ion source device 101 of another example. Hereinafter, a description will be given on only the differences from the ion source device 1 of Embodiment 1.

In the ion source device 1 of Embodiment 1, in the gas supply unit 26, each of the three pulse-control gas valves (21A, 21B, and 21C) is provided on each of the gas supply pipes (24A, 24B, and 24C) of the oxygen gas supply system, the methane gas supply system and the helium gas supply system.

On the other hand, in the ion source device 101 of this example, in the gas supply unit 126, one pulse-control gas valve 21 is provided on a gas introduction pipe 125 at one end of which a gas nozzle 19 is provided.

Therefore, the number of components is smaller, and the device is smaller, and it is easy for the ion source control device 31 to control the pulse-control gas valve 21. That is, in the ion source device 1 of the first embodiment, it is necessary to change the pulse-control gas valves to be operated (21A, 21B, and 21C) depending on the type of the gas to be supplied; however, in the ion source device 101 of this example, only one pulse-control gas valve 21 has to be operated regardless of the type of the gas to be used.

The present invention is not limited only to the configuration of the above-described embodiments, and it is possible to obtain many aspects of embodiment.

For example, although the synchrotron using the pre-stage accelerator 3 and the main accelerator 4 is used as the accelerator in the above-described embodiments, the accelerator is not limited to the above synchrotron, and an appropriate accelerator can be used, such as a cyclotron using only a circular accelerator. Also in this case, an arbitrary type of gas can be supplied in a pulsed manner into the plasma chamber 12 to obtain desired ions, and the same action and effect as in the above-described embodiments can be achieved.

### INDUSTRIAL APPLICABILITY

The present invention can be applied to a particle beam generation device which emits charged particles.

### REFERENCE SIGNS LIST

- 1:: Ion source device
- 2:: Ion separation device
- 3:: Pre-stage accelerator
- 4:: Main accelerator
- 5:: Injection device
- 6:: Deflection electromagnet
- 7:: Acceleration cavity
- 8:: Emission device
- 9:: Irradiation device
- 10:: ECR ion source
- 11:: Plasma
- 12:: Plasma chamber
- 13:: Electromagnet
- 14:: Electrode
- 15:: Ion beam
- 16:: Vacuum pump
- 17A, 17B, 17C:: Gas cylinder
- 19:: Gas nozzle
- 20:: Microwave waveguide
- 21, 21A, 21B, 21C:: Pulse-control gas valve
- 22A, 22B, 22C:: Shut-off gas valve
- 23A, 23B, 23C:: Flow-rate-adjustment gas valve
- 24A, 24B, 24C:: Gas supply pipe
- 25:: Gas introduction pipe
- 26:: Gas supply unit
- 30:: Control device
- 31:: Ion source control device
- 32:: Accelerator control device
- 33:: Irradiation control device
- 100:: Particle beam generation device

## Claims

1. An ion source device which generates an ion beam from a gas introduced into a vacuum, the ion source device comprising:
a chamber whose inside is a vacuum;
a gas supply unit which supplies a gas to the inside of the chamber; and
an ionization energy supply unit which supplies ionization energy for ionizing atoms of the gas to the inside of the chamber,
wherein the gas supply unit includes:
a plurality of gas cylinders each of which is filled with each of a plurality types of gases;
a gas passage connected to the gas cylinders to feed the gases to the chamber; and
a pulse-control gas valve which is attached to the gas passage and is settable to an open state in a pulsed manner for a short period of time.

2. The ion source device according to claim 1, wherein the ionization energy supply unit includes an ionization energy supply-period control unit which controls, depending on a desired ion species, a period on a gas amount changing curve to which an ionization energy supply period is set, and on the gas amount changing curve, a gas amount in the chamber changes due to an opening operation and a closing operation of the pulse-control gas valve, and in the ionization energy supply period the ionization energy is supplied into the chamber by the ionization energy supply unit.

3. The ion source device according to claim 2, wherein the ionization energy supply-period control unit also performs control in such a manner that the ionization energy supply period in which the ionization energy is supplied is set to a period after the pulse-control gas valve transitions from an open state to a closed state.

4. A particle beam generation device comprising:
an ion source device which generates an ion beam;
an ion separation device which removes other ions than target ions from the ion beam generated by the ion source device;
a main accelerator which accelerates and emits the ion beam from which the other ions than the target ions have been removed by the ion separation device; and
an irradiation device which irradiates a target with a high-energy ion beam emitted from the main accelerator,
wherein the ion source device is the ion source device according to claim 1, 2, or 3, and the ion source device includes:
an ion species change request transmission unit that requests to change ion species to be emitted from the irradiation device; and
a gas valve opening/closing control unit which controls, following the request to change ion species transmitted by the ion species change request transmission unit, opening and closing of the pulse-control gas valve to supply the gas into the chamber from a part of the plurality of gas cylinders of the ion source device.

5. The particle beam generation device according to claim 4, wherein
the main accelerator performs four modes of operation which include an acceleration mode in which ions are accelerated, an emission mode in which the accelerated ions are emitted, a deceleration mode in which deceleration is performed, and an injection mode in which a next acceleration mode is waited for,
the request to change ion species by the ion species change request transmission unit is performed during the emission mode, the deceleration mode, or the injection mode, and
generation of ions by the ion source device is performed in the injection mode.

6. A ion beam generation method for generating an ion beam from a gas introduced by an ion source device into a vacuum, wherein the ion source device includes: a chamber whose inside is a vacuum; a gas supply unit which supplies gas to the inside of the chamber; and an ionization energy supply unit which supplies ionization energy for ionizing atoms of the gas to the inside of the chamber, and the gas supply unit includes: a plurality of gas cylinders each of which is filled with each of a plurality types of gases; a gas passage connected to the gas cylinders to feed the gases; and a pulse-control gas valve which is attached to the gas passage and is settable to an open state in a pulsed manner for a short period of time, the ion beam generation method comprising:
causing a pulse-control gas valve corresponding a part of gas cylinder of the plurality of gas cylinders filled with the different types of gases to operate to introduce the gas into the chamber in a pulsed manner.
